# EUROPEAN PATENT APPLICATION

(11) **EP 4 541 183 A1**
(43) Date of publication of application: **23.04.2025**
(21) Application number: 24204499.8
(22) Date of filing: 03.10.2024
(51) Int. Cl.: A01K 29/00, A61N 5/06

(54) **RADIATION DEVICE FOR ANIMALS, PREFERABLY CATTLE, TO INCREASE MILK PRODUCTION, AND ANIMAL RADIATION METHOD USING SUCH A DEVICE**

(30) Priority: 16.10.2023 SI 202300136
(71) Applicant: Krusic, Ziga, 71332 Waiblingen (DE); Staric, Joze, 1000 Ljubljana (SI); Jankovec, Marko, Polhov Gradec (SI)
(72) Inventor: KRUSIC, Ziga, 71332 Waiblingen (DE); STARIC, Joze, 1000 Ljubljana (SI); JANKOVEC, Marko, Polhov Gradec (SI); KRUSIC, Joze, 1000 Ljubljana (SI); HODNIK, Jaka, 1000 Ljubljana (SI); MITTERHOFER, Stefan, 39020 Partschins, Bolzano (IT)
(74) Representative: Golmajer Zima, Marjanca

(57) **Abstract**

An irradiation device (100) for animals, preferably cattle, comprising a platelike section (1) formed of at least one flat element (5), preferably a plurality of flat elements, and lighting units (2) arranged on the bottom side of the flat element (5), said lighting units being the source of ultraviolet light having a wavelength of 280 nm to 315 nm.

## Description

### Subject of invention

The subject of the present invention is an irradiation device for animals, preferably cattle, to increase milk production, and an animal irradiation method using such a device.

### Technical problem

The technical problem is to configure an irradiation device for animals, preferably cattle, to increase milk production in intensive stall-fed dairy cattle, where the animals are not exposed to sunlight, wherein the use of such a device will neither interfere with the established process of cattle rearing and milk collection nor adversely affect the animals, preferably cattle. The device for the animals should be configured to be economically viable.

### Prior art

The vitamin D group, in particular vitamin D₃, belongs to essential vitamins that affect the functioning of various biological functions of living beings, including animals, like cattle. In cattle, vitamin D affects milk production. In the developed world, especially in the temperate zone, the cattle are mostly reared in stables and do not have regular direct exposure to the sun and the consequent cutaneous synthesis of vitamin D. Even if cattle have access to pasture, cutaneous synthesis of vitamin D may not be possible throughout the year. Cattle intended for milk production are therefore often dependent on vitamin D added to the feed. Vitamin D supplementation is problematic for several aspects. When preparing a ration for cows, it is virtually impossible to dose the optimum amount of vitamin D for each animal because the ration is prepared by mixing several ingredients for all or most animals, feed consumption varies from animal to animal, and the persistence of vitamin D in feed varies over time. In addition, the amount of vitamin D required by an individual animal is not constant but varies and depends on the condition of the animal, such as physiological state, milk production. In cattle, a change in blood vitamin D concentration, in particular a decrease can be detected in early lactation due to the increased calcium requirement for milk production and the reduced feed intake before and after calving. Therefore, despite vitamin D supplementation, the amount of vitamin D in the feed may be insufficient. Vitamin D hypervitaminosis can also occur in animals when a certain constant amount of vitamin D is supplemented or if too much vitamin D is supplemented for too long. Excessive vitamin D supplementation rarely adversely affects the health of cattle because the animal excretes it in its faeces. However, vitamin D overdose certainly has a negative economic impact. The blood concentration of vitamin D can be monitored by regular blood measurements, but this is not sustainable in the animal for several reasons. First of all, because blood collection is stressful for the animal, blood can only be taken by trained persons, and measuring vitamin D in the blood represents an additional cost.

Adding vitamin D to feed is further problematic in organic breeding systems, which are obligated to use only organically produced feed additives. These are expensive and therefore have a negative impact on production costs.

When cattle spend most of the day on pasture throughout the year, they are exposed to natural sunlight. Sunlight irradiating the animal's skin with ultraviolet light causes vitamin D to be produced in the animal's body. When there is enough natural sunlight, the animal's body produces as much vitamin D as it needs. The natural process of vitamin D formation does not result in an excessive amount of vitamin D being formed in the animal's body, so hypervitaminosis does not occur. Vitamin D is synthesised in the animal when irradiated with sunlight independently of its daily feed intake.

Research has been carried out in which animals, especially cattle, were irradiated with ultraviolet light from an artificial source. The results showed that UV light irradiation has a positive effect on the production of vitamin D in the animals, and thus on milk production.

### Solution to the technical problem

The relative terms such as below, above, in front of, behind are defined according to the orientation of the device in the operative position, when the device is arranged above the animal's back and correspond to the animal's orientation when it is located below the device.

The technical problem is solved by the irradiation device for animals, preferably cattle, to increase milk production, the main characteristics of which are defined in the first independent claim.

The irradiation device for animals, preferably cattle, to increase milk production, which results in production of an amount of vitamin D in animals, preferably cattle, comprises a platelike section consisting of at least one flat element, preferably a plurality of flat elements, and lighting units arranged on the bottom side of the flat element, said lighting units being the source of ultraviolet light having a wavelength of 280 nm to 315 nm. A respective flat element is formed either as a flat plate or as a longitudinally curved plate having a radius substantially corresponding to the animal's back curvature when the device is in the operative position. The flat elements are spaced apart from each other so as to form an air gap between them which allows warm air to escape from the area of the device and thus the space within the area of the device to be cooled. The flat elements are preferably arranged to be spaced apart in the longitudinal direction. The flat element may also be in the form of a slat. The size of the device is adapted to the size of an average animal, preferably cattle, to be irradiated. So, the length and width of the device are adapted to an average length and width of the animal, preferably cattle, to be irradiated.

A respective platelike section is divided into a front portion, central portion and rear portion which have variable densities of lighting units. The lengthwise distance between the lighting units in the central portion is larger than the lengthwise distance between the lighting units of the front and/or rear portions. The lengthwise distance between the lighting units in the central portion is substantially twice the lengthwise distance between the lighting units of the front and/or rear portions. The lighting units may also be equidistantly distributed over the platelike section, resulting in a uniform density of the distributed lighting units, which, however, does not ensure a uniform animal irradiation.

The lighting units arranged on a respective flat element are LEDs, halogen lamps or fluorescent lamps emitting light of 280 nm to 315 nm, preferably emitting light of 290 nm to 300 nm.

The device is made of a material having good thermal conductivity and low specific density such as aluminium and/or aluminium alloy.

The device may comprise a control device controlling the operation of the lighting units.

A method of irradiating animals, preferably cattle, using such an irradiation device comprises the following steps:
a) detecting an animal present within an irradiation area, preferably a milking robot milking parlour, by an electronic identification unit attached to the animal;
c) switching on the irradiation device and thus the irradiation lighting units;
e) switching off the irradiation device after a predetermined irradiation time, at the latest when the milking device is switched off.

The method may comprise additional steps:
b) verifying the irradiation status data and comparing same against the predetermined data on the required irradiation for a respective animal stored either in the control unit or a separate memory unit that is part of the control unit, step b) being performed after step a) and before step c);
d) determining irradiation time and irradiation intensity based on the comparison against the previously input data, step d) being performed after step c) and before step e);
f) inputting the animal irradiation sessions and irradiation parameters in the memory unit, step f) being performed after step e).

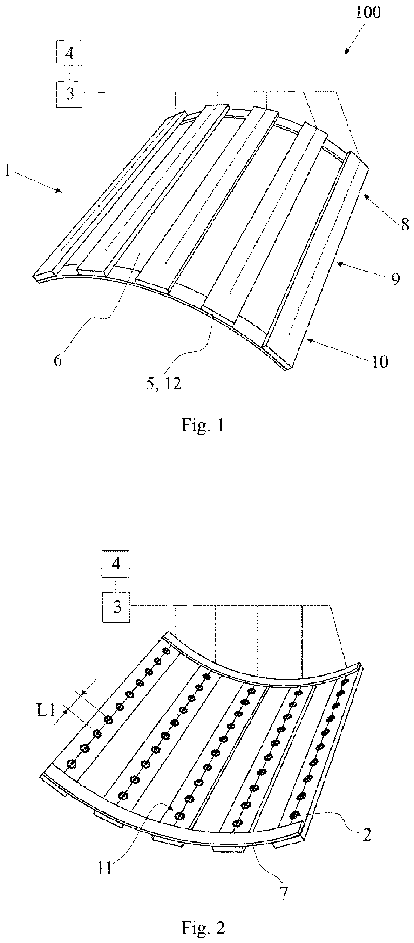

The irradiation device of the invention for animals, preferably cattle, will be described hereinbelow in more detail by way of an embodiment and drawings representing in
- Fig. 1: device of the invention comprising a plurality of slat-like flat elements, top view
- Fig. 2: device of the invention comprising a plurality of slat-like flat elements, bottom view

A device 100 for irradiation of animals, preferably cattle, to increase milk production and enable sufficient vitamin D to be produced naturally, comprises
- a platelike section 1 formed of at least one flat element 5, preferably a plurality of flat elements, and
- lighting units 2 arranged on the bottom side of a respective flat element 5 and being a light source with a wavelength in the ultraviolet range, i.e. a wavelength of 280 nm to 315 nm. The device 100 may optionally also comprise a control unit 3 for controlling the lighting units 2 connected to a respecting lighting unit 2, and a power supply unit 4.

The platelike section 1 comprises a front portion 8, a central portion 9 and a rear portion 10 and has a length and width substantially corresponding to the length and width of the device 100. The length and width of the device 100 are adapted to an average length and width of the animal, preferably cattle, to be irradiated. In the embodiment, the length of the device 100 is in the range of 1000-1500 mm, the width of the device is in the range of 500-800 mm. As such, the device, when in use, is arranged substantially over the entire animal's body, which is not a limitation for the device to have other dimensions.

A respective flat element 5 is formed as a flat plate. When the platelike section 1 consist of more than one flat element 5, these are spaced apart, preferably equidistantly spaced. An air gap 6 is thus formed between the flat elements. The flat elements 5 are fastened to at least one carrying element 7.

The lighting units 2, which are a light source with a wavelength in the ultraviolet range, are arranged on the bottom side of a respective flat element 5. The lighting units may be LEDs, halogen lamps or fluorescent lamps emitting light of a wavelength between 280 nm and 315 nm, preferably 295 nm, in which the efficiency of photolysis of 7-dehydroxycholesterol to cholecalciferol, i.e. vitamin D formation, is highest. The lighting units 2 are arranged on the bottom side 11 of a respective flat element 5 at a distance L1 from each other, said distance being uniform along a respective flat element 5 and thus equal along the platelike section 1, or the distance L1 is non-uniform resulting in a varying density of distribution of lighting units 2 on the flat element 5 and thus on the platelike section 1. In order to achieve a more uniform irradiation of an animal over its entire upper body, the distance L1 between the lighting units 2 on the flat element 5 is non-uniform, such that the density of the lighting units 2 along the flat element 5 varies as a function of the distance of the flat element 5 from the animal being irradiated. In case of the non-uniform distance L1, the lighting units 2 are distributed in a way that the distance between the lighting units 2 in the central portion 9 of the platelike section 1 is larger than the distance between the lighting units 2 in the front 8 and rear portions 10 of the platelike section 1. The widthwise distance between the respective lighting units 2 is identical, while the lengthwise distance between the respective lighting units 2 depends on the portion in which a lighting unit 2 is located. In the front portion 8 and the rear portion 10 of the platelike section 1, the lighting units 2 are distributed at a uniform distance L1, while in the central portion 9, they are distributed at a uniform distance which is twice the distance L1 between the lighting units 2 of the front 8 and/or rear portions 10. When the device 100 is in operation, the front 8 and rear portions 10 of the platelike section 1 are located above the animal's front body part and the rear body part which is rather uneven and rounded. A denser distribution of lighting units 2 in these two portions 8, 10 ensures that the required amount of light energy is received in the form of UV light in both the animal's front body part and the rear body part having a rather uneven surface. A varying density of distribution of lighting units 2 on the platelike section 1 allows the animal to receive an adequate amount of energy and uniform irradiation over its upper body part, while at the same time preventing the formation of burns on the animal's skin, which would be caused in particular by a more intense irradiation of a smaller area of the skin.

In order to yield optimum UV light irradiation over the animal's entire upper body part, the platelike section 1 is curved in the longitudinal direction, the curvature radius of the platelike section 1 being such to follow as closely as possible the curvature of the back of the animal, preferably cattle, when the device 100 is in the operating position, i.e. above the animal. This ensures that the entire device 100 is evenly spaced from the animal's body and that the animal is irradiated more evenly. The curved platelike section 1 consists of at least one flat element 5 which is curved with a radius of the platelike section in the longitudinal direction or of a plurality of flat elements 5 which are spaced from each other in the longitudinal direction and inclined with respect to the horizontal plane to follow the curvature of the platelike section 1.

The lighting units 2 distributed on the flat elements 5 are connected to each other in series and powered by an energy source. The operation of the lighting units 2 and thus the entire device 100 is controlled by the control unit 3 which is a constituent part of the device.

In the embodiment, the flat element 5 is formed as a slat 12, the number of slats 12 being more than one, preferably there is an even number of slats 12. The slats 12 are distributed in the platelike section 1 equidistantly in the longitudinal direction. An air gap 6 is formed between the slats, which gap allows the hot air to be evacuated from the area of the device 100. The slats 12 are inclined towards each other in a mirrorlike manner with respect to the horizontal plane. The length of a respective slat 12 is identical to the length of the platelike section 1 and thus the length of the device 100. The bottom side 11 of a respective slat 12 is provided with lighting units 2 which are distributed in the front 8 and rear portions 10 of a respective slat 12 at a distance L1, while they are distributed in the central portion 9 at a distance from each other that is twice the distance L1.

The device is made of a material having good thermal conductivity and low specific density such as aluminium and/or aluminium alloy. In its size, the device corresponds to the size of an average animal to be irradiated.

The device 100 is arranged at a certain height at a substantially horizontal distance from the ground in the milking robot parlour which is intended for milking the animals. Such a parlour is limited in size and does not allow the animal to move, which, in consequence, allows a uniform irradiation of the animal during the entire operating time of the device. The device 100 is arranged at a height that is horizontally spaced from the ground in a way that a respective platelike section 1 is at a distance of 200-500 mm, preferably 300-400 mm from the animal. Such an arrangement of the device 100 enables safe irradiation of the animal with ultraviolet light without any danger of the animal getting burns, while at the same time providing adequate irradiation with ultraviolet light, which results in the production of a sufficient amount of vitamin D that an animal needs for the optimum milk production. At the same time, the arrangement of the device 100 at such a height allows the animal to easily enter and exit the milking parlour. The device 100 may be arranged in the milking parlour also by means of a dedicated robotic device that enables a real-time arrangement of the device 100 to/from the operating position.

Each animal to be milked using a milking device (milking robot) is equipped with its electronic identification unit, e.g. an RFID bracelet, which serves to identify each animal. When an animal enters the milking parlour, the control unit 3 as a constituent part of the device 100 identifies the animal via the electronic identification unit, checks the irradiation status and compares it against the irradiation schedule for the respective animal. Based on the irradiation schedule, the control unit 3 switches the device 100 on and irradiation of the animal starts. The irradiation is carried out following a predetermined schedule and method which comprises irradiation intensity and irradiation time and depends on an individual animal. The irradiation time never exceeds the milking time. To this purpose, the control unit 3 is connected to the milking device (not subject of the present invention) which communicates to the control unit that milking is over and the control unit 3 switches off the device 100. When milking is over, the animal is no longer irradiated and can exit the milking parlour. The control unit 3 saves the data on the irradiation time of an individual animal to the memory and uses them in the computation in the next milking. If the previous milking of the animal was sufficiently long for the animal to have received the foreseen daily intake of ultraviolet radiation, the irradiation is terminated for that day. Otherwise, when the animal comes in for the next milking on the same day, it is irradiated for the time needed to produce the adequate amounts of vitamin D. The time an animal spends in the milking parlour in a day is longer than the time needed to irradiate the animal with UV light to produce the daily amount of vitamin D needed for optimum milk production. At the same time, over-irradiation which might cause skin burns of the animal is also prevented. It is not possible to overdose an animal with vitamin D produced by UV irradiation because the animal's body only produces the amount of vitamin D it needs. Due to the known amount of irradiation needed to produce a sufficient amount of vitamin D, which the animal receives regularly on a daily basis, the animal is not deficient in vitamin D.

## Claims

1. An irradiation device (100) for animals, preferably cattle, to increase milk production, **characterized by** comprising
- a platelike section (1) comprising a front portion (8), a central portion (9) and a rear portion (10) and formed of at least one flat element (5), preferably a plurality of flat elements, and
- lighting units (2) arranged on the bottom side (11) of the flat element (5) and being a source of ultraviolet light with a wavelength of 280 nm to 315 nm.

2. The device according to claim 1, **characterized in that** a respective flat element (5) is formed either as a flat plate or as a longitudinally curved plate having a radius substantially corresponding to the animal's back curvature when the device (100) is in the operative position.

3. The device according to any preceding claim, **characterized in that** the flat elements (5) are spaced apart from each other so as to form an air gap (6) between them.

4. The device according to claim 3, **characterized in that** the flat elements (5) are spaced apart from each other in the longitudinal direction.

5. The device according to any preceding claim, **characterized by** a varying density of the lighting units (2) arranged on a respective platelike section (1) formedof at least one flat element (5), preferably a plurality of flat elements.

6. The device according to claim 5, **characterized in that** the lengthwise distance (L1) between the lighting units (2) in the central portion (9) of the platelike section is larger than the lengthwise distance (L1) between the lighting units (2) in the front (8) and/or rear portions (10) of the platelike section.

7. The device according to claim 6, **characterized in that** the lengthwise distance (L1) between the lighting units (2) in the central portion (9) of the platelike section is substantially twice the lengthwise distance (L1) between the lighting units (2) in the front (8) and/or rear portions (10) of the platelike section.

8. The device according to any preceding claim, **characterized in that** the lighting units (2) arranged on a respective flat element (5) are LEDs, halogen lamps or fluorescent lamps emitting light of 280 nm to 315 nm, preferably of 290 nm to 300 nm.

9. The device according to any preceding claim, **characterized in that** the flat element (5) is formed as a slat (12).

10. The device according to any preceding claim, **characterized by** comprising a control device (3) controlling the operation of the lighting units (2).

11. The device according to any preceding claim 1-4, 8-10, **characterized by** a uniform density of the lighting units (2) arranged on a respective flat element (5).

12. The device according to any preceding claim, **characterized by** being made of a material having good thermal conductivity and low specific density, preferably aluminium and/or aluminium alloys.

13. The device according to any preceding claim, **characterized in that** the length and width of the device are adapted to an average length and width of the animal, preferably cattle, to be irradiated.

14. A method of irradiating animals, preferably cattle, using the device (100) according to any preceding claim, comprising the following consecutive steps:
a) detecting an animal present within an irradiation area, preferably a milking robot milking parlour, by an electronic identification unit attached to the animal;
c) switching on the device (100) and thus the irradiation lighting units (2), preferably when the milking robot is switched on;
e) switching off the device (100) after a predetermined irradiation time, preferably when the milking robot is switched off.

15. The method according to the preceding claim, **characterized by** comprising further steps:
b) verifying the irradiation status data and comparing same against the predetermined data on the required irradiation for a respective animal stored either in the control unit or a separate memory unit that is part of the control unit, step b) being performed after step a) and before step c);
d) determining irradiation time and irradiation intensity based on the comparison against the previously input data, step d) being performed after step c) and before step e);
f) inputting the animal irradiation sessions and irradiation parameters in the memory unit, step f) being performed after step e).
